# EUROPÄISCHE PATENTSCHRIFT

(11) **EP 0 362 528 B1**
(45) Veröffentlichungstag und Bekanntmachung des Hinweises auf die Patenterteilung: **01.03.1995**
(21) Anmeldenummer: 89115205.0
(22) Anmeldetag: 17.08.1989
(51) Int. Cl.: A61F 5/37

(54) **Abduktionskissen**
Abduction pillow
Coussin d'abduction

(30) Priorität: 18.08.1988 DE 3827981
(43) Veröffentlichungstag der Anmeldung: 11.04.1990
(73) Patentinhaber: GMT Gesellschaft für medizinische Technik mbH, D-22767 Hamburg (DE)
(72) Erfinder: Engelbrecht, Eckart, Dr. med., D-2000 Hamburg 60 (DE)
(74) Vertreter: Heldt, Gert, Dr. Dipl.-Ing.

(56) Entgegenhaltungen:
- DE-A- 3 517 343
- DE-U- 8 616 180
- US-A- 4 598 701

## Beschreibung

Die Erfindung betrifft ein Kissen zum Abstützen eines ruhigzustellenden Armes auf einem Kissenkörper (49), der mindestens eine den Arm unterstützende Auflagefläche (1) aufweist, wobei mindestens eine Seitenwandung (50) des Kissenkörpers (49) quer zur Auflagefläche (1) verläuft und als eine Körperausnehmung (59) ausgebildet ist, die jeweils von einer im Tragezustand oberen und einer unteren Körperkante begrenzt ist.

Derartige Kissen werden insbesondere verwendet, wenn bei Verletzungen im Bereich des Schultergelenkes dieses von durch das Gewicht des Armes verursachten Belastungen entlastet werden soll. Die Kissen weisen im allgemeinen eine den Arm unterstützende Auflagefläche auf und sind in unterschiedlichen Ausführungsformen bekannt geworden.

Aus der DE-A-35 17 343 ist ein Abduktionskissen bekannt, dessen Kissenkörper eine den Arm unterstützende Auflagefläche aufweist, wobei eine Seitenwandung des Kissenkörpers quer zur Auflagefläche verläuft. Diese in lotrechter Richtung verlaufende Seitenwandung weist eine konkave Körperausnehmung auf. Um ein gutes Anliegen des Kissenkörpers, bzw. seiner Seitenwandung, an der Körperseite des Patienten zu erreichen, muß dieser Kissenkörper aus einem sehr weichen, elastischen Material bestehen. Dieses wirkt sich nachteilig auf die Stabilität des Kissenkörpers und insbesondere seiner Auflagefläche aus. Ein weicher Werkstoff von ausreichender Stabilität der Auflagefläche ermöglicht zwar eine gute Anpassung an den Körper eines Patienten, besitzt aber keine dem menschlichen Körper fest zugeordnete Lage, beispielsweise beim Gehen und Drehen des menschlichen Körpers, da eine konkave Körperausnehmung dem menschlichen Körper nicht ausreichend angepaßt ist.

Aufgabe der vorliegenden Erfindung ist es daher, ein Kissen der einleitend genannten Art so zu verbessern, daß das Gewicht von Kissen und Arm großflächig auf den Körper des Patienten übertragen wird und den Tragekomfort und den Kissensitz zu verbessern.

Diese Aufgabe wird erfindungsgemäß dadurch gelöst, daß die Oberkante der Auflagefläche im Bereich der oberen Körperkante verläuft, wobei sich die Auflagefläche von ihrer Oberkante in einer von der Körperausnehmung weggewandten Richtung in einer geneigten Ebene schräg abwärts in Richtung auf eine sie begrenzende Unterkante erstreckt, die gemeinsam mit der unteren Körperkante eine Bodenfläche begrenzt, und daß die Körperausnehmung aus Oberflächen mindestens zweier Zylinder zusammengesetzt ist, deren Achsen etwa in lotrechter Richtung verlaufen und die voneinander verschiedene Krümmungen aufweisen, wobei eine einen kleineren Durchmesser aufweisende Zylinderoberfläche mit einer Taillenwölbung versehen ist und eine einen größeren Durchmesser aufweisende Zylinderoberfläche mit einer Bauchwölbung versehen ist.

Dieses Kissen bzw. dessen Körperausnehmung der Seitenwandung ist der Anatomie des menschlichen Körpers weitgehend angepaßt. Die Körperausnehmung weist einen größeren Durchmesser, der der Bauchwölbung des Patienten, und einen kleineren Durchmesser auf, der der Taillenwölbung des Patienten entspricht. Diese Ausbilung des Kissenkörpers ermöglicht eine sichere Fixierung am Körper des Patienten und überträgt das Gewicht von Kissen und Arm großflächig auf den Körper des Patienten, wodurch der Tragekomfort erheblich verbessert wird. Das Kissen besitzt eine dem menschlichen Körper fest zugeordnete Lage sowohl beim Gehen und Drehen des menschlichen Körpers. Es kann beispielsweise aus Schaumstoff hergestellt werden, dessen Festigkeit eine ausreichenden Stabilität des Kissens ermöglicht. .

Durch die Neigung der Auflagefläche wird der Arm in einer Lage unterstützt, in der sich das Schultergelenk in einer mittleren Drehstellung von 30 Grad bis 45 Grad gegenüber einem am Körper herabhängenden Arm befindet. Diese Lage ist für eine Ausheilung des Schultergelenkes besonders günstig. Darüber hinaus ist eine weitgehende Entlastung der im Bereich der Schulter und des Rükkens angeordneten Bänder, Sehnen und Muskeln gewährleistet. Dadurch wird der Arm in einer Lage unterstützt, in der er eine Ruhigstellung für einen großen Zeitraum verträgt, ohne daß Schmerzen auftreten. Die Anordung der Auflagefläche ermöglicht darüber hinaus auch die Aufnahme von Kräften, die nicht ausschließlich in lotrechter Richtung verlaufen. Auch in der liegenden Position wird der Arm ausreichend von der Auflagefläche unterstützt. Gleichzeitig nimmt die Auflagefläche auch die bei Geh- und Drehbewegungen auftretenden Kräfte auf.

Gemäß einer weiteren bevorzugten Ausführungsform der Erfindung weist das Kissen längeneinstellbare elastische Gurte auf, die einerseits das Kissen am Körper des Patienten haltern und andererseits den abzustützenden Arm auf der Auflagefläche fixieren. Aufgrund ihrer elastischen Eigenschaften und durch eine relativ große Breite ist gewährleistet, daß die Gurte am menschlichen Körper keine Schnüreinwirkungen hinterlassen und sich trotzdem an unterschiedliche Bewegungssituationen anpassen können. Durch Längenanpassbarkeit der Gurte kann das Kissen an unterschiedliche anatomische Gegebenheiten des Patienten angepasst werden und ein beschwerdefreies Tragen gewährleistet sein. Durch die Fixierung des abzustützenden Armes auf der Auflagefläche mittels längenanpassbarer Haltegurte kann darüber hinaus auch bei unterschiedlichen Armdicken und Körpergrößen eine Befestigung erzielt werden, die weder zu fest ausgebildet ist noch die Gefahr eines Herausrutschen des Armes in sich birgt.

Das Kissen ist mit Hilfe der Gurte leicht montierbar und kann je nach den individuellen Bedürfnissen des Patienten hinsichtlich seines Sitzes nachkorrigiet werden. Der Patient selbst kann derartige Nachkorrekturen vornehmen. Andererseits sitzt das Kissen am Körper des Patienten so fest, daß es auch in der Rückenlage nicht in Richtung auf den Rücken des Patienten wegrutschen kann. Es kann sehr verschiedenen Sitz- und Liegepositionen sowie Gangsituationen genau angepasst werden. Schließlich ist es möglich, durch einfaches Lösen der den Arm auf der Auflagefläche haltenden Armgurte den Arm in die Lage zu versetzen, Bewegungen auszuführen, die er in Form von vorgeschriebenen Übungen zu seiner Ausheilung benötigt.

Weitere Einzelheiten der Erfindung ergeben sich aus der nachfolgend ausführlichen Beschreibung und den beigefügten Zeichnungen, in denen bevorzugte Ausführungsformen der Erfindung beispielsweise veranschaulicht sind.

In den Zeichnungen zeigen:

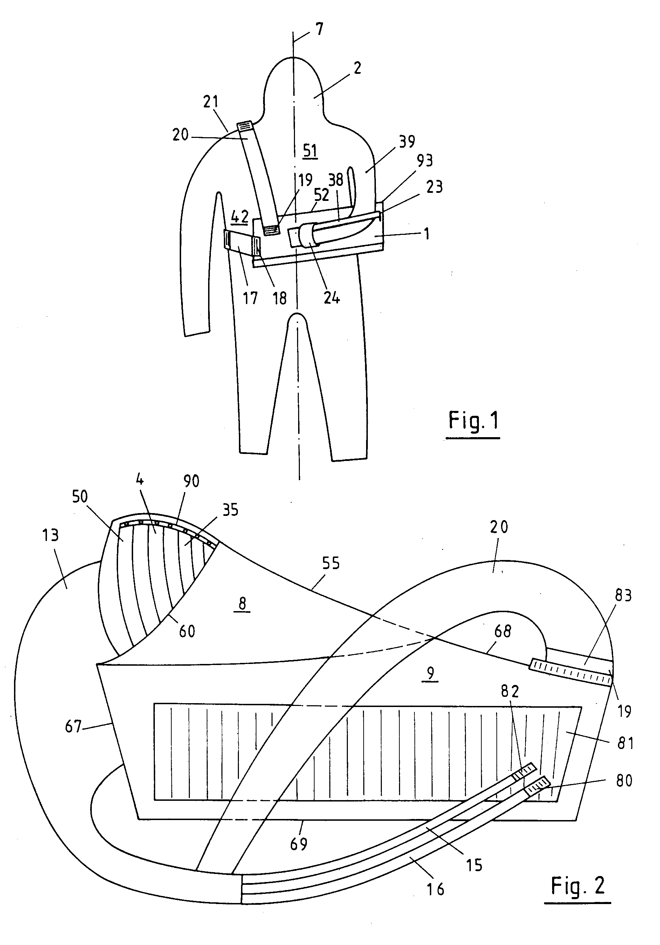

- Fig. 1:: Eine Vorderansicht eines Patienten mit einem umgeschnallten Kissen, das den linken Arm abstützt,
- Fig. 2:: ein Blick vor eine Stirnfläche eines Kissens zur Abstützung eines linken Armes,

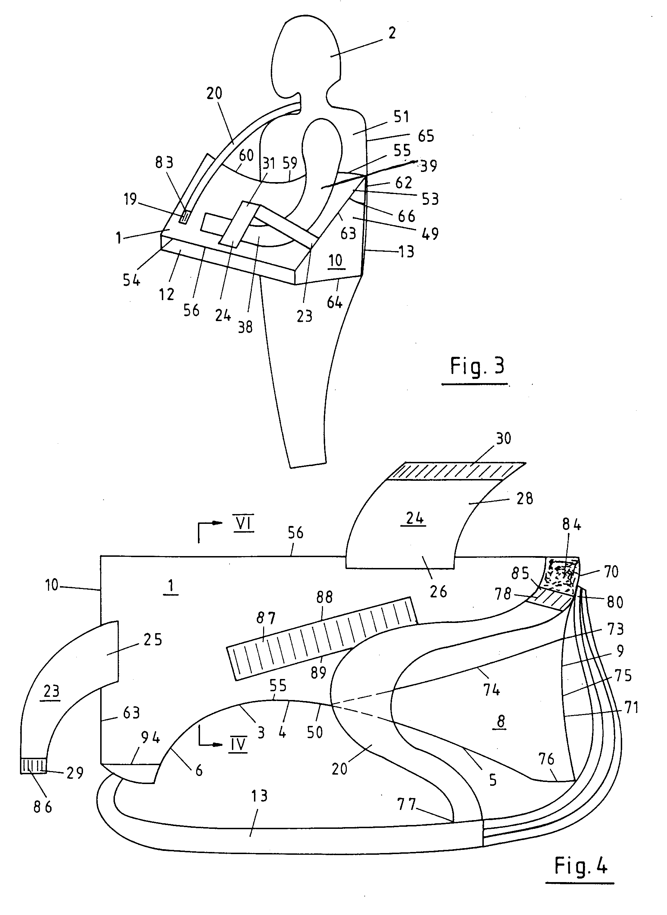

- Fig. 3:: eine Seitenansicht eines Patienten mit einem umgeschnallten Kissen zur Abstützung des linken Armes,
- Fig. 4:: eine Draufsicht auf eine Auflagefläche eines Kissens zur Abstützung eines linken Armes,

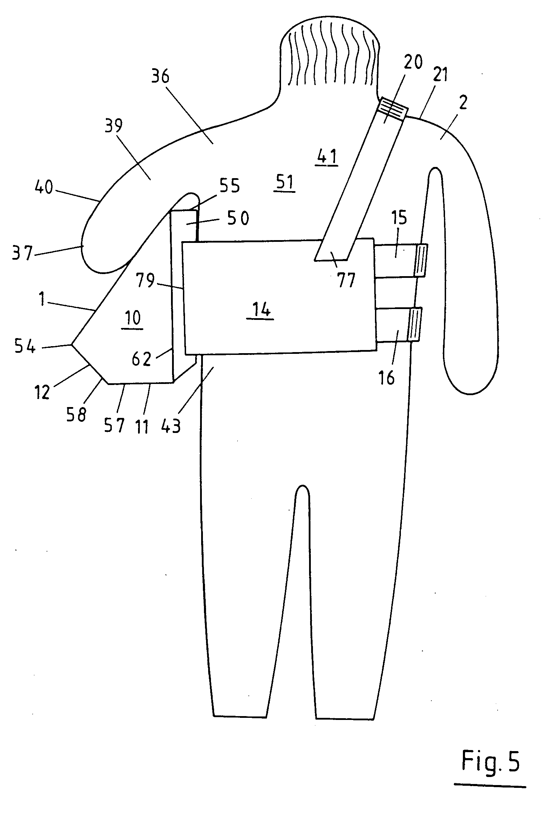

- Fig. 5:: eine Rückansicht eines Patienten mit umgeschnalltem Kissen, das den linken Arm unterstützt,

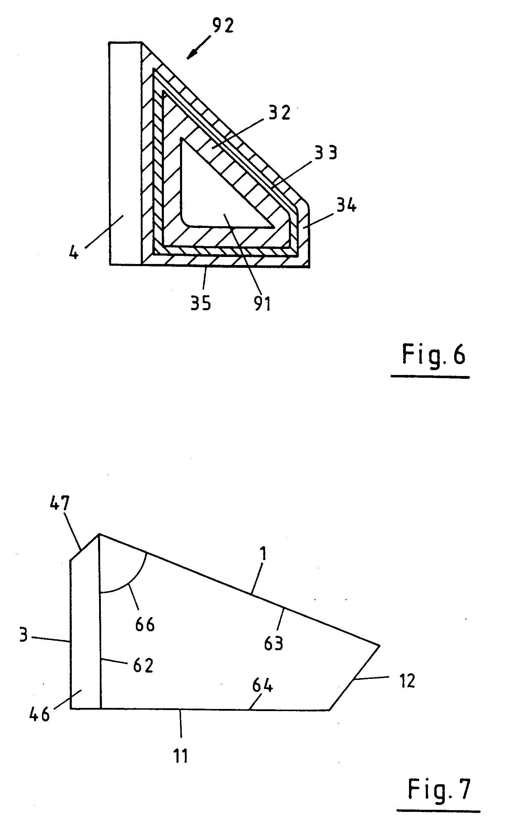

- Fig. 6:: einen Querschnitt durch ein Kissen gemäß der Schnittlinie VI - VI in Fig. 4,
- Fig. 7:: eine Seitenansicht eines Kissens vor eine hintere Stirnfläche,

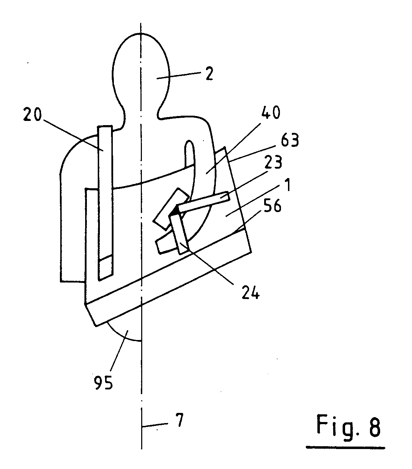

- Fig. 8:: eine Teilvorderansicht eines ein Kissen tragenden Patienten.

Ein Kissen besteht im wesentlichen aus einem Kissenkörper (49) und Gurten (13, 20, 23, 24), die an dem Kissenkörper (49) befestigt sind. Der Kissenkörper (49) liegt mit einer Seitenwandung (50) an einem Körper (51) eines Patienten (2). Zu diesem Zwecke ist die Seitenwandung in Form einer Abstützfläche (3) ausgebildet, die sich mit einer Wölbung (4) dem Körper (51) des Patienten (2) im Bereich seines Bauches (52) und seiner Taille (53) anpasst. In entsprechender Weise ist die Bauchwölbung (4) in eine dem Bauch zugewandte Bauchwölbung (5) und eine der Taille zugewandte Taillenwölbung unterteilt. Die Taillenwölbung (6) geht stetig in die Bauchwölbung (5) über. Sowohl die Taillenwölbung (6) als auch die Bauchwölbung (5) sind als Oberflächen jeweils eines Zylinders ausgebildet, von denen der die Taillenwölbung (6) ausbildende Zylinder einen kleineren Radius besitzt als der die Bauchwölbung (5) ausbildende Zylinder. Der Radius des die Bauchwölbung (5) ausbildenden Zylinders liegt im Bereich zwischen 15 cm bis 20 cm und ist vorzugsweise etwa 32 cm lang. Der Radius des die Taillenwölbung ausbildenden Zylinders liegt im Bereich zwischen 10 cm und 20 cm und ist vorzugsweise 15 cm lang.

Die Seitenwandung (50) verläuft im wesentlichen quer zu einer Auflagefläche (1) , auf der ein Arm (40) des Patienten (2) abgestützt ist. Diese Auflagefläche (1) besitzt eine Oberkante (55), zwischen der und einer ihr gegenüberliegenden Unterkante (56) sich die Auflagefläche (1) erstreckt. Dabei ist die Oberkante (55) dem Körper (51) des Patienten (2) benachbart, während die Unterkante (56) eine Bodenfläche (11) des Kissenkörpers (49) im Bereich einer körperfernen Region des Kissenkörpers (49) begrenzt. Die Bodenfläche (11) kann aus zwei Teilflächen bestehen, von denen eine erste Teilfläche (57) einer von der Seitenwandung (50) gebildeten Körperausnehmung (59) zugewandt ist, während eine zweite Teilfläche (58) in einem stumpfen Winkel aufwärts in Richtung auf die Auflagefläche (1) geneigt ist und eine Seitenfläche (12) bildet.

Die Oberkante (55) verläuft im Bereich einer oberen Körperkante (60), die die Körperausnehmung (59) in ihrem oberen Teil begrenzt. Von der oberen Körperkante (60) erstreckt sich die Körperausnehmung in Richtung auf eine untere Körperkante, entlang der die Bodenfläche (11) in die Seitenwandung (50) des Kissenkörpers (49) übergeht. In einem hinteren, der Taillenwölbung (6) benachbarten Bereich der Oberkante (55) bildet diese die obere Körperkante (60) aus. Im Bereich des Übergangs von der Taillenwölbung (6) in die Bauchwölbung (5) verläuft die obere Körperkante (60) körpernah am Körper (51) des Patienten (2), während die Oberkante (55) von der Auflagefläche (1) eine Abschlußfläche (8) abgrenzt, deren Neigung gegenüber der Seitenwandung (50) geringer als die der Auflagefläche (1) ist.

Die Taillenwölbung (6) endet an einer der Bauchwölbung abgewandten Hinterkante (62) des Kissenkörpers (49). Von dieser Hinterkante (62) aus gesehen verläuft etwa ein Drittel der oberen Körperkante (60) im Bereich der Taillenwölbung (6), die sodann stetig in die Bauchwölbung (5) einmündet. Die Hinterkante (62) begrenzt eine hintere Stirnfläche (10), die mit der Auflagefläche (1) eine hintere Querkante (63) bildet. Darüber hinaus wird die hintere Stirnfläche (10) von einer hinteren Unterkante (64) begrenzt, die sie gemeinsam mit der Bodenfläche (11) ausbildet.

Die Hinterkante (62) ragt über eine Ebene nicht hinaus, die von einem Rücken (65) des Patienten (2) aufgespannt wird. Auf diese Weise ist es möglich, daß der Patient (2) auch in seiner Rückenlage seinen Arm (40) auf der Auflagefläche (1) abstützen kann, ohne daß der Kissenkörper (49) seine Lage verändern muß, wenn sich der Patient (2) in seine Rückenlage begibt.

Die Neigung der Auflagefläche (1) gegenüber der Seitenwandung (50) ergibt sich aufgrund der Größe eines Winkels (66), der von der hinteren Querkante (63) einerseits und der Hinterkante (62) andererseits eingeschlossen wird. Dieser Winkel liegt im Bereich zwischen 20 Grad und 70 Grad und besitzt vorzugsweise einen Wert von 45 Grad.

Die hintere Stirnfläche (10) verläuft in einer etwa lotrechten Ebene, die in etwa senkrecht auf einer von der Auflagefläche (1) aufgespannten Ebene steht. Auf diese Weise ist dafür gesorgt, daß auch die Hinterkante (62) in etwa lotrecht verläuft.

Die Auflagefläche (1) wird auf ihrer der hinteren Stirnfläche (10) gegenüber liegenden Seite von einer vorderen Stirnfläche (9) begrenzt. Diese vordere Stirnfläche (9) wird einerseits von einer Vorderkante (67) begrenzt, die das Ende der Seitenwandung (50) auf deren der Hinterkante (62) abgewandten Seite bildet. Darüber hinaus wird die vordere Stirnfläche (9) von einer die Auflagefläche (1) begrenzenden vorderen Querkante (68) sowie von einer die Bodenfläche (11) vorne begrenzenden vorderen Unterkante (69) begrenzt. Die vordere Stirnfläche (9) kann im Bereich der die Auflagefläche (1) begrenzenden Unterkante (54) zunächst in einer ersten Teilfläche (70) etwa planparallel zur hinteren Stirnfläche (10) in Richtung auf die Seitenwandung (5o) verlaufen. Nach etwa einem Drittel der Länge der vorderen Querkante (68) kann die vordere Stirnfläche (9) eine Neigung in eine von der hinteren Stirnfläche (10) abgewandte Richtung aufweisen und eine zweite Teilfläche (71) ausbilden. Die Neigung zwischen der ersten Teilfläche (70) und der zweiten Teilfläche (71) kann einem Steigungswinkel entsprechen, der etwa zwischen 10 Grad und 20 Grad liegt und im Regelfall 15 Grad beträgt.

In Richtung auf eine von der ersten Teilfläche (70) und der zweiten Teilfläche (71) gebildete Schneidkante (73) läuft ein von der oberen Körperkante (60) im Bereich der Bauchwölbung (5) abweichender Teil (74) der Oberkante (55). Dieser abweichende Teil (74) begrenzt mit der im Bereich der Bauchwölbung (5) verlaufenden oberen Körperkante (60) und einer im Bereich der zweiten Teilfläche (71) verlaufenden oberen Teilkante (75) die Abschlußfläche (8), die gegenüber der Auflagefläche (1) eine in Richtung auf die Bodenfläche (11) gerichtete Neigung besitzt. Diese Neigung kann einem Neigungswinkel entsprechen, der zwischen 15 und 30 Grad liegt und bevorzugt 22 Grad aufweist.

Die obere Körperkante (60) kann in einem Bereich, in dem sie in die vordere Querkante (68) übergeht, einen Abschnitt (76) aufweisen, der die Bauchwölbung (5) verläßt und in etwa parallel zur Unterkante (54) der Auflagefläche (1) verläuft. Dieser Abschnitt (76) begrenzt eine als lotrechte Ebene ausgebildete Abschnittsfläche, die einen stumpfen Winkel mit der Bauchwölbung (5) ausbildet.

Das Kissen weist mindestens einen Schultergurt (20) auf, der im Bereich eines ersten Endes (77) in einen Bauchgurt einmündet und im Bereich seines anderen Endes (78) mit dem Kissenkörper (49) verbunden ist. Der Schultergurt (20) ist lösbar mit dem Kissenkörper (49) und fest mit dem Bauchgurt (13) verbunden. Der Bauchgurt (13) verläuft im wesentlichen in einer horizontalen Ebene und umspannt den Bauch (52) des Patienten (2). Der Bauchgurt (13) mündet in die Seitenwandung (50) im Bereich der Taillenwölbung (6) ein und ist fest mit dieser entlang einer Linie (79) verbunden, die in etwa parallel zur Hinterkante (62) verläuft. Mit seinem lösbaren Ende (80) ist der Bauchgurt (13) auf einem Klettverschluß (81) befestigt, der sich über die vordere Stirnfläche (9) erstreckt. Zu diesem Zwecke ist am lösbaren Ende (80) des Bauchgurtes (13) ein Gegenstück (82) des Klettverschlusses (81) befestigt. Der Klettverschluß (81) erstreckt sich großflächig über die vordere Stirnfläche (9), um einen möglichst großen Bezirk zur Verfügung zu haben, an dem je nach individuellen Bedürfnissen des Patienten (2) die Gegenstücke mit dem Klettverschlusß (81) verbunden werden können.

Der Bauchgurt (13) besteht im wesentlichen aus zwei Teilen, von denen ein breitflächiger Rückenteil (14) entlang der Linie (79) in die Taillenwölbung (6) einmündet. An diesen Rückenteil (14) schließt sich ein Taillenteil an, der aus zwei einander etwa parallel verlaufendne Teilgurten (15, 16) besteht. Der Schultergurt (20) ist in einem Bereich am Bauchgurt (13) befestigt, in dem der Rückenteil (14) in die beiden Teilgurte (15, 16) übergeht. An dieser Stelle mündet der Schultergurt etwa senkrecht in den in horizontaler Ebene verlaufenden Bauchgurt (13) ein und ist fest mit diesem verbunden. Von dort erstreckt sich der Schultergurt (20) über eine vom abgestützten Arm (40) abgewandte Schulter (21) des Patienten (2). Darüber hinaus ist es auch möglich, daß die Teilgurte (15, 16) an ihrem dem Rückenteil (14) abgewandten losen Enden in einen Abschlußgurt (17) übergehen, der mit einem Bauchgurtverschluß (18) an der vorderen Stirnfläche (9) befestigt ist. Außer einem Klettverschluß (81) kommen alle anderen denkbaren Verschlüsse, Knopf-, Reiß- oder Haftverschlüsse in Betracht.

Der Schultergurt (20) ist an seinem dem Rückenteil (14) abgewandten losen Ende (83) mit der Auflagefläche (1) einzeln lösbar verbunden. Zu diesem Zwecke erstreckt sich über die Auflagefläche (1) entlang der vorderen Querkante (68) ein Schultergurtverschluß (19). Dieser Schultergurtverschluß (19) kann als ein Klettverschluß (84) ausgebildet sein, der sich großflächig entlang der vorderen Stirnfläche (9) erstreckt. Am losen Ende (83) ist ein Gegenstück (85) für den Klettverschluß (84) vorgesehen, das je nach den individuellen Maßen des Patienten (2) auf dem großflächigen Klettverschluß (84) befestigt werden kann. Jede andere Möglichkeit zur Ausbildung des Schultergurtverschlusses (9) ist möglich, z.B. als Knopf-, Reiß- und Haftverschluß.

Sowohl der Schultergurt (20) als auch der Rückenteil (19) des Bauchgurtes (13) sind breitflächig ausgebildet, um in den Gurten (13, 20) auftretende Kräfte möglichst großflächig auf den Körper (51) des Patienenten (2) zu übertragen. Auf diese Weise werden hohe Flächenpressungen und damit Schmerzerscheinungen vermieden.

Auf der Auflagefläche (1) ist eine den Arm (40) aufnehmende Schiene angeordnet. Diese Schiene besteht aus mindestens einem den Arm (40) fixierenden Gurt (23, 24). Zweckmäßigerweise verlaufen zwei Gurte (23, 24) in ihren Richtungen etwa senkrecht zueinander. Dabei verläuft ein Oberarmgurt (23) in etwa senkrecht zur hinteren Querkante (63) und ein Unterarmgurt (24) in etwa senkrecht zur Unterkante (54). Der Oberarmgurt ist mit seinem festen Ende im Bereich einer der Unterkante (54) zugewandten unteren Hälfte der Querkante (63) befestigt und verläuft in etwa parallel dazu in deren unmittelbare Nachbarschaft. Er besitzt ein von der hinteren Querkante (63) über die Auflagefläche (1) reichendes loses Ende (86), das mit einer sich auf der Auflagefläche (1) erstreckenden Kupplung (87) verbunden werden kann. Zu diesem Zwecke ist die Kupplung (87) als ein sich breitflächig über die Auflagefläche (1) erstreckender Klettverschluß ausgebildet, auf dem ein Gegenstück (29) haftet, das am losen Ende (86) des Oberarmgurtes (23) befestigt ist.

Der Unterarmgurt (24) ist mit einem festen Ende (26) im Bereich der der hinteren Querkante (62) zugewandten Hälfte der Unterkante (56) befestigt. Das feste Ende (26) verläuft in etwa parallel zur Unterkante (56)in deren unmittelbarer Nachbarschaft. Der Armgurt (24) ist mit einem dem festen Ende (26) abgewandten losen Ende (28) an einer auf der Auflagefläche (1) vorgesehenen Kupplung angekoppelt. Diese Kupplung erstreckt sich über den Mittelteil der Auflagefläche (1). Zweckmäßigerweise ist der Unterarmgurt (24) an dem breitflächigen Klettverschluß (84) angekoppelt, der auch zur Ankoppelung des Oberarmgurtes (23) dient. Zu diesem Zwecke erstreckt sich der Klettverschluß (87) über die Auflagefläche (1) als ein breitflächiger Streifen, dessen einander etwa parallel verlaufenden Längskanten (88, 89) sich in etwa von der ersten Teilfläche (70) der vorderen Stirnfläche (9) in Richtung auf die Hinterkante (62) schräg über die Auflagefläche (1) erstrecken. Auf diesem Klettverschluß der Kupplung (87) haftet sowohl das Gegenstück (29) des Oberarmgurtes (23) als auch ein Gegenstück (30) des Unterarmgurtes (24). Um eine Vielzahl von Variationsmöglichkeiten der Ankopplung zu haben, kann mindestens eines der Gegenstücke (29, 3o) auf beiden Seiten des Oberarmgurtes (23) bzw. Unterarmgurtes (24) ausgebildet sein. Auf diese Weise ist es möglich, eines der beiden Gegenstücke (29) auf dem Klettverschluß der Kupplung (7, 8) zu befestigen und das andere Gegenstück (30) auf dem Ende (29) des Oberarmgurtes (23) anhaften zu lassen.

Zweckmäßigerweise sind die Gurte (13, 15, 16, 2o, 23, 24) elastisch ausgebildet. Auf diese Weise behindern sie den Patienten (2) nur wenig in seiner Bewegungsfreiheit. Außerdem verursachen sie keine Schmerzgefühle.

Das Kissen kann aus einem einheitlichen Material ausgebildet sein. Es ist auch möglich, einen Kern (32) zu formen und diesen mit einer Hülle (33) zu umgeben. Die Hülle (33) sollte aus einem feuchtigkeitsbeständigen Material ausgebildet sein. Sie ist zweckmäßigerweise aus einem Kunststoff hergestellt. Darüber hinaus kann die Hülle (33) mindestens bereichsweise von einer Bespannung (34) umgeben sein. Diese Bespannung (34) ist als ein Gewebe (35) ausgebildet. Dieses Gewebe ist mindestens im Bereich der Bauchwölbung (5) feuchtigkeitsaufsaugend ausgebildet. Es kann mit Hilfe eines Verschlusses (19)an der Seitenwandung (50) befestigt sein. Als Verschluß kommt ein Klettverschluß oder ein Reißverschluß in Betracht, die beide das bequeme Tragen des Kissens nicht beeinträchtigen dürfen.

Der Kern (32) ist zweckmäßigerweise aus einem Material mit geringen spezifischem Gewicht ausgebildet. In erster Linie kommt ein weich elastischer Schaumstoff in Betracht, der sich den jeweiligen Maßen des Patienten (2) am besten anpassen kann. Es ist auch möglich, andere geschäumte Kunststoffe zu verwenden. Darüber hinaus kann der Kern auch als ein einen Innenraum (91) umschließender Hohlkörper (92) ausgebildet sein.

Im Bereich des Überganges von der oberen Körperkante (60) in die hintere Querkante (63) kann von der Auflagefläche (1) eine der Hinterkante (62) zugewandte Spitze (93) abgeschnitten sein. Diese steht möglicherweise einem Oberarm (39) im Wege. Im Bereich der abgeschnittenen Spitze (93) begrenzt eine Verbindungsfläche (47) den Kissenkörper (49).Diese Verbindungsfläche (47) weist im Bereich eines kurzen Stückes (94) der Oberkante (55) eine Neigung gegenüber der Auflagefläche (1) auf, die zwischen 60 Grad und 90 Grad liegt. Zweckmäßigerweise besitzt die Verbindungsfläche (47) eine Neigung von 65 Grad gegenüber der Auflagefläche (1).

Die Auflagefläche (1) spannt eine Ebene auf, die gegenüber einer sich in Schrittrichtung des Patienten (2) erstreckenden lotrechten Ebene (7) geneigt ist. Diese Ebene der Auflagefläche (1) schließt mit der in Schrittrichtung des Patienten (2) verlaufenden Ebene einen Winkel ein, der zwischen 30 und 60 Grad beträgt. Vorzugsweise besitzt dieser Winkel eine Größe von 45 Grad.

Zur Abstützung des Armes (40) eines Patienten (2) wird diesem zunächst der Schultergurt (20) umgelegt und dieser mit seinem Schultergurtverschluß (19) die Traghöhe des Kissenkörpers (49) festlegend mit der Auflagefläche (1) verbunden. Anschließend wird der Bauchgurt (13) dem Patienten (2) um seinen Rücken (41) herumgelegt und die beiden Teilgurte (15, 16) mit ihren dem Rückenteil (14) abgewandten Enden am Klettverschluß (81) befestigt. Je nach der Körperausbildung des Patienten (2) werden die lösbaren Enden (80) auf einem Teil des Klettverschlusses (81) befestigt, das mehr oder minder weit von der Vorderkante (67) der Seitenwandung (50) entfernt ist.

Nach dem Festschnallen des Kissens liegt dieses im Bereich der Wölbung (4) dicht am Körper (51) des Patienten (2) an. Sein Bauch (42) wird von der Bauchwölbung (5), seine Taille (43) von der Taillenwölbung (6) aufgenommen.

Nunmehr wird der Arm (40) auf der Auflagefläche (1) befestigt, indem sein Unterarm (38) von dem Unterarmgurt (24) und sein Oberarm (39) von dem Oberarmgurt (23) umfaßt wird. Dabei verläuft der Oberarm (39) etwa parallel zur hinteren Querkante (63) und der Unterarm (38) etwa parallel zur Unterkante (54). In dieser Lage besitzt das Schultergelenk eine mittlere Drehstellung gegenüber dem herabhängenden Arm (40) von etwa 30 Grad bis 45 Grad. Der Arm (40) ist damit in einer anatomisch sehr günstigen Stellung fixiert. Außerdem sind den Arm (40) tragende Bänder und Muskeln entlastet. Die Längenanpassbarkeit der Gurte (23, 24) erlaubt deren Anpassung an unterschiedlich ausgebildete Arme, insbesondere bei individuell verschiedenen Armdicken. Dabei werden einerseits Quetschbelastungen vermieden und andererseits auch dünne Arme sehr genau fixiert.

Je nach der Genesung des Patientens (2) und der Art der Schädigung des Schultergelenkes kann durch Lösen des Unterarmgurtes (23)und des Oberarmgurtes (24) nach Wahl des behandelnden Arztes ein vorgebbarer Bewegungsspielraum realisiert werden.

Die Stirnfläche (10) verläuft in einer gemeinsamen Ebene mit dem Rücken (41) des Patienten (2). Hierdurch wird auch in einer Rückenlage des Patienten (2) der Arm (40) in der vorgegebenen Lage genau fixiert, ohne daß sich Verschiebungen im Schultergelenk (36) ergeben. Damit wird der Patient (2) auch im wesentlichen vor Schmerzen bewahrt.

Während einer Gehbewegung eines Patienten (2) werden dynamische Belastungen durch die Elastizität der Gurte (13, 20, 23, 24) gedämpft. Damit wird auch das Schultergelenk (36) gegen wechselnden Druck- und Zugbelastungen geschützt.

Von besonderer Bedeutung ist die Anbringung des Kissens am Körper (51) des Patienten (2). Eine von der Auflagefläche (1) aufgespannte Ebene ist gegenüber einer sich in Schrittrichtung des Patienten (2) erstreckenden lotrechten Ebene (7) geneigt. Die Neigung der von der Auflagefläche (1) aufgespannten Ebene gegenüber der sich in Schrittrichtung des Patienten (2) erstreckenden Schrittebene beträgt etwa 30 bis 60 Grad und vorzugsweise etwa 45 Grad. Auf diese Weise ist auch der Unterarm (38) in Schrittrichtung schräg nach vorne ausgerichtet und hat damit eine optimale Ruheposition. Darüber hinaus kann für den Arm (40) dadurch eine sehr gute Lage erzielt werden, daß die einzelnen Gurte (13, 20, 23, 24) in ihrer Länge den Bedürfnissen des Patienten (2) sehr genau angepasst werden können.

## Patentansprüche

1. Kissen zum Abstützen eines ruhigzustellenden Armes auf einem Kissenkörper (49), der mindestens eine den Arm unterstützende Auflagefläche (1) aufweist, wobei mindestens eine Seitenwandung (5O) des Kissenkörpers (49) quer zur Auflagefläche (1) verläuft und als eine Körperausnehmung (59) ausgebildet ist, die jeweils von einer im Tragezustand oberen und einer unteren Körperkante begrenzt ist, dadurch gekennzeichnet, daß die Oberkante (55) der Auflagefläche (1) im Bereich der oberen Körperkante (60) verläuft, wobei sich die Auflagefläche (1) von ihrer Oberkante (55) in einer von der Körperausnehmung (59) weggewandten Richtung in einer geneigten Ebene schräg abwärts in Richtung auf eine sie begrenzende Unterkante (54) erstreckt, die gemeinsam mit der unteren Körperkante eine Bodenfläche (11) begrenzt, und daß die Körperausnehmung (59) aus Oberflächen mindestens zweier Zylinder zusammengesetzt ist, deren Achsen im Tragezustand etwa in lotrechter Richtung verlaufen und die voneinander verschiedene Krümmungen aufweisen, wobei eine einen kleineren Durchmesser aufweisende Zylinderoberfläche mit einer Taillenwölbung (6) versehen ist und eine einen größeren Durchmesser aufweisende Zylinderoberfläche mit einer Bauchwölbung (5) versehen ist.

2. Kissen nach Anspruch 1, dadurch gekennzeichnet, daß die Oberkante (55) mindestens einen Teil der oberen Körperkante (60) bildet.

3. Kissen nach Anspruch 1 oder 2, dadurch gekennzeichnet, daß die Unterkante (54) die Bodenfläche (11) auf einer der Körperausnehmung (59) abgewandten Seite begrenzt.

4. Kissen nach einem der Ansprüche 1 bis 3, dadurch gekennzeichnet, daß die Bodenfläche (11) eben ist.

5. Kissen nach einem der Ansprüche 1 bis 3, dadurch gekennzeichnet, daß die Bodenfläche (11) aus mindestens zwei Teilflächen (57, 58) besteht, von denen eine der Körperausnehmung (59) zugewandte erste Teilfläche (57) etwa horizontal verläuft und die sich anschließende zweite Teilfläche (58) körperfern angeordnet ist und in einem stumpfen Winkel in Richtung auf die Auflagefläche (1) aufwärts geneigt ist.

6. Kissen nach einem der Ansprüche 1 bis 4, dadurch gekennzeichnet, daß die Oberkante (55) die obere Körperkante (6O) in einem im Tragezustand hinteren Teil der Körperausnehmung (59) bildet.

7. Kissen nach einem der Ansprüche 1 bis 6, dadurch gekennzeichnet, daß die Körperausnehmung (59) in ihrem im Tragezustand hinteren Teil von der Oberfläche des Zylinders mit kleinerem Durchmesser und in ihrem vorderen Teil mit der Oberfläche des Zylinders mit größerem Durchmesser begrenzt ist.

8. Kissen nach einem der Ansprüche 1 bis 7, dadurch gekennzeichnet, daß der im Tragezustand hintere Teil der Körperausnehmung (59) unter stetiger Vergrößerung des Radius ihrer Krümmung in den vorderen Teil übergeht.

9. Kissen nach einem der Ansprüche 1 bis 8, dadurch gekennzeichnet, daß die Taillenwölbung (6) an einer der Bauchwölbung (5) abgewandten Hinterkante (62) endet, und die obere Körperkante (6O) von der Hinterkante (62) etwa ein Drittel ihrer Länge im Bereich der Taillenwölbung (6) verläuft und danach stetig in die Bauchwölbung (5) mündet.

10. Kissen nach einem der Ansprüche 1 bis 9, dadurch gekennzeichnet, daß die Hinterkante (62) eine im Tragezustand hintere Stirnfläche (1O) begrenzt, die mit der Auflagefläche (1) eine im Tragezustand hintere Querkante (63) und mit der Bodenfläche (11) eine im Tragezustand hintere Unterkante (64) bildet.

11. Kissen nach einem der Ansprüche 1 bis 10, dadurch gekennzeichnet, daß die hintere Querkante (63) und die Hinterkante (59) einen Winkel im Bereich zwischen 2O Grad und 7O Grad einschließen.

12. Kissen nach Anspruch 10 oder 11, dadurch gekennzeichnet, daß die Auflagefläche (1) auf ihrer der im Tragezustand hinteren Stirnfläche (1O) gegenüberliegenden Seite von einer im Tragezustand vorderen Stirnfläche (9) begrenzt wird, die von einer der Taillenwölbung (6) abgewandten Vorderkante (67) der Bauchwölbung (5), von einer die Auflagefläche (1) vorne begrenzenden vorderen Querkante (68) und von einer die Bodenfläche (11) vorne begrenzenden vorderen Unterkante (54) begrenzt ist, daß die vordere Stirnfläche (9) im Bereich der die Auflagefläche (1) begrenzenden Unterkante (54) zunächst in einer ersten Teilfläche (7O) etwa planparallel zur hinteren Stirnfläche (1O) in Richtung auf die Bauchwölbung (5) verläuft und etwa nach einem Drittel der vorderen Querkante (68) eine Neigung in eine von der hinteren Stirnfläche (1O) abgewandten Richtung aufweist und eine zweite Teilfläche (71) bildet, daß die Oberkante (55) im Bereich der Bauchwölbung (5) in Richtung einer von dieser abweichenden Geraden verläuft, die sich vom Übergang der Taillenwölbung (6) in die Bauchwölbung (5) in Richtung auf jene Stelle der vorderen Querkante (68) erstreckt, an der die erste Teilfläche (7O) in die zweite Teilfläche (71) übergeht, daß die Gerade mit einer die zweite Teilfläche (58) begrenzenden oberen Teilkante der vorderen Querkante (68) und einem von der Geraden im Bereich der Bauchwölbung (5) von der oberen Körperkante (6O) abgetrennten Teilbereich eine Abschlußfläche (8) begrenzt, die gegenüber der Auflagefläche (1) in Richtung auf die Bodenfläche (11) abwärts geneigt ist und daß die obere Teilkante gegenüber einer sich in Richtung auf die Unterkante (54) der Auflagefläche (1) erstreckenden unteren Teilkante der vorderen Querkante (68) eine Neigung von etwa 15 Grad bis 3O Grad besitzt.

13. Kissen nach Anspruch 12, dadurch gekennzeichnet, daß die Neigung 22 Grad beträgt.

14. Kissen nach einem der Ansprüche 1 bis 13, dadurch gekennzeichnet, daß die Bauchwölbung (5) einen Radius im Bereich von etwa 15 cm bis 2O cm besitzt.

15. Kissen nach Anspruch 14, dadurch gekennzeichnet, daß der Radius der Bauchwölbung etwa 32 cm ist.

16. Kissen nach einem der Ansprüche 1 bis 15, dadurch gekennzeichnet, daß die Taillenwölbung (6) einen Radius im Bereich zwischen etwa 1O cm bis 2O cm besitzt.

17. Kissen nach Anspruch 16, dadurch gekennzeichnet, daß der Radius der Taillenwölbung (6) etwa 15 cm beträgt.

18. Kissen nach einem der Ansprüche 1 bis 17, dadurch gekennzeichnet, daß es mindestens einen Schultergurt (2O) aufweist, der im Bereich eines ersten Endes (77) in einen Bauchgurt (3O) einmündet und im Bereich seines anderen Endes (83) mit dem Kissenkörper (49) verbunden ist.

19. Kissen nach Anspruch 18, dadurch gekennzeichnet, daß der Schultergurt (2O) lösbar mit einer der Flächen (1, 9, 1O) des Kissenkörpers (49) und fest mit dem Bauchgurt (13) verbunden ist.

20. Kissen nach einem der Ansprüche 18 oder 19, dadurch gekennzeichnet, daß der Bauchgurt (13) im wesentlichen in einer horizontalen Ebene verläuft.

21. Kissen nach Anspruch 20, dadurch gekennzeichnet, daß der Bauchgurt (13) in die Taillenwölbung (6) einmündet und fest mit dieser entlang einer Linie (79) verbunden ist, die etwa parallel zur Hinterkante (62) verläuft und lösbar an der im Tragezustand vorderen Stirnfläche (9) befestigt ist.

22. Kissen nach Anspruch 21, dadurch gekennzeichnet, daß der Bauchgurt (13) an der ersten Teilfläche (7O) der vorderen Stirnfläche (9) über einen sich großflächig auf der ersten Teilfläche (7O) erstreckenden Klettverschluß (81) befestigt ist.

23. Kissen nach einem der Ansprüche 18 bis 22, dadurch gekennzeichnet, daß der Schultergurt (2O) im Bereich des Übergangs vom Rückenteil (14) in den Taillenteil in etwa senkrechter Richtung in den Rückenteil (14) einmündet.

24. Kissen nach einem der Ansprüche 18 bis 23, dadurch gekennzeichnet, daß der Schultergurt (2O) mit der Auflagefläche (1) lösbar verbunden ist.

25. Kissen nach Anspruch 24, dadurch gekennzeichnet, daß sich auf der Auflagefläche (1) entlang der vorderen Querkante (68) eine das lösbare Ende (8O) des Schultergurtes (2O) aufnehmende Kupplung erstreckt.

26. Kissen nach einem der Ansprüche 23 bis 25, dadurch gekennzeichnet, daß der Schultergurt (2O) sich über eine Schulter (21) erstreckt, die einem auf der Auflagefläche (1) abgestützten Arm (4O) abgewandt ist.

27. Kissen nach einem der Ansprüche 23 bis 26, dadurch gekennzeichnet, daß der Schultergurt (2O) breitflächig ausgestaltet ist.

28. Kissen nach einem der Ansprüche 1 bis 27, dadurch gekennzeichnet, daß auf der Auflagefläche (1) eine den Arm (4O) aufnehmende Schiene angeordnet ist.

29. Kissen nach Anspruch 28, dadurch gekennzeichnet, daß die Schiene aus mindestens einem den Arm (4O) fixierenden Gurt (23, 24) besteht.

30. Kissen nach Anspruch 28 oder 29, dadurch gekennzeichnet, daß auf der Auflagefläche (1) zwei etwa senkrecht zueinander verlaufende Gurte (23, 24,) angeordnet sind.

31. Kissen nach einem der Ansprüche 28 bis 30, dadurch gekennzeichnet, daß ein Oberarmgurt (23) sich in etwa senkrecht zur hinteren Querkante (63) und ein Unterarmgurt (24) sich in etwa senkrecht zur Unterkante (54) jeweils über die Auflagefläche (1) erstrecken.

32. Kissen nach Anspruch 31, dadurch gekennzeichnet, daß der Oberarmgurt (23) mit einem festen Ende, das in etwa parallel zur hinteren Querkante (63) verläuft, im Bereich einer der Unterkante (54) zugewandten unteren Hälfte der hinteren Querkante (63) in deren unmittelbaren Nachbarschaft auf der Auflagefläche (1) befestigt ist.

33. Kissen nach Anspruch 32, dadurch gekennzeichnet, daß der Oberarmgurt (23) mit einem dem festen Ende abgewandten losen Ende (86) an eine Kupplung (87) angekoppelt ist, die in einem zwischen der oberen Körperkante (6O) und der Unterkante (54) liegenden Mittelteil der Auflagefläche (1) vorgesehen ist.

34. Kissen nach einem der Ansprüche 31 bis 33, dadurch gekennzeichnet, daß der Unterarmgurt (24) mit einem festen Ende (26), das in etwa parallel zur Unterkante (54) der Auflagefläche (l) verläuft, im Bereich einer der hinteren Querkante (63) zugewandten Hälfte der Unterkante (54) in deren unmittelbaren Nachbarschaft auf der Auflagefläche (1) befestigt ist.

35. Kissen nach Anspruch 34, dadurch gekennzeichnet, daß der Unterarmgurt (24) mit einem dem festen Ende (26) abgewandten losen Ende (28) an eine Kupplung (87) angekoppelt ist, die im Mittelteil der Auflagefläche (1) vorgesehen ist.

36. Kissen nach einem der Ansprüche 33 bis 35, dadurch gekennzeichnet, daß der Unterarmgurt (24) an dem die Kupplung (87) bildenden breitflächigen Klettverschluß des Oberarmgurtes (23) angekoppelt ist.

37. Kissen nach einem der Ansprüche 33 bis 35, dadurch gekennzeichnet, daß der breitflächige Klettverschluß als ein sich quer über die Auflagefläche (1) erstreckender Streifen ausgebildet ist, dessen etwa parallel zueinander verlaufende Längskanten (88, 89) sich etwa in einer Richtung von der ersten Teilfläche (7O) zur Hinterkante (62) erstrecken.

38. Kissen nach einem der Ansprüche 32 bis 37, dadurch gekennzeichnet, daß von den losen Enden (28, 86,) des Oberarmgurtes (23) bzw. Unterarmgurtes (24) mindestens eines sowohl auf seiner dem auf der Auflagefläche (1) befestigten Klettverschluß zugewandten Unterseite als auch auf seiner dieser gegenüber liegenden Oberseite mit einem Klettverschluß versehen ist, von denen ein unterer auf dem auf der Auflagefläche (1) befestigten Klettverschluß und ein oberer auf dem am jeweils anderen losen Ende (28, 86) befestigten Klettverschluß aufliegt.

39. Kissen nach einem der Ansprüche 18 bis 38, dadurch gekennzeichnet, daß die Gurte (13, 2O, 23, 24) elastisch ausgebildet sind.

40. Kissen nach einem der Ansprüche 1 bis 39, dadurch gekennzeichnet, daß der Kissenkörper (49) aus einem einheitlichen Material ausgebildet ist.

41. Kissen nach einem der Ansprüche 1 bis 40, dadurch gekennzeichnet, daß der Kissenkörper (49) aus einem Kern (32) und einer den Kern (32) umgebenden Hülle (33) ausgebildet ist.

42. Kissen nach Anspruch 41, dadurch gekennzeichnet, daß die Hülle (33) feuchtigkeitsbeständig ausgebildet ist.

43. Kissen nach Anspruch 42, dadurch gekennzeichnet, daß die Hülle (33) mindestens bereichsweise von einer Bespannung (34) umgeben ist.

44. Kissen nach Anspruch 43, dadurch gekennzeichnet, daß die Bespannung (34) als Gewebe (35) ausgebildet ist.

45. Kissen nach Anspruch 44, dadurch gekennzeichnet, daß das Gewebe (35) mindestens im Bereich der Bauchwölbung (5) feuchtigkeitsaufsaugend ausgebildet ist.

46. Kissen nach einem der Ansprüche 41 bis 45, dadurch gekennzeichnet, daß der Kern (32) aus einem Material mit geringem spezifischen Gewicht ausgebildet ist.

47. Kissen nach Anspruch 46, dadurch gekennzeichnet, daß der Kern (32) als einen Innenraum (91) umschließender Hohlkörper (92) ausgebildet ist.

48. Kissen nach einem der Ansprüche 10 bis 47, dadurch gekennzeichnet, daß im Bereich des Übergangs der oberen Körperkante (6O) in die hintere Querkante (63) von der Auflagefläche (1) eine der Hinterkante (62) zugewandte Spitze (92) abgeschnitten ist.

## Claims

1. A cushion for supporting an arm to be rested on a cushion body (49) which has at beast one support surface (1) for supporting the arm underneath same, wherein at least one side wall (50) of the cushion body (49) extends transversely relative to the support surface (1) and is in the form of a body recess (59) which is respectively defined by body edges which are upper and lower edges in the condition in which the cushion is being worn, characterised in that the upper edge (55) of the support surface (1) extends in the region of the upper body edge (60), the support surface (1) extending from its upper edge (55) in a direction away from the body recess (59) in an inclined plane obliquely downwardly towards a lower edge (54) defining it, which lower edge together with the lower body edge defines a bottom surface (11), and that the body recess (59) is composed of surfaces of at least two cylinders whose axes extend approximately in a perpendicular direction when the cushion is being worn and which have curvatures that are different from each other, wherein a cylinder surface which is of smaller diameter is provided with a waist curve (6) and a cylinder surface having a larger diameter is provided with an abdominal curve (5).

2. A cushion according to claim 1 characterised in that the upper edge (55) forms at least a part of the upper body edge (60).

3. A cushion according to claim 1 or claim 2 characterised in that the lower edge (54) defines the bottom surface (11) on a side that is remote from the body recess (59).

4. A cushion according to one of claims 1 to 3 characterised in that the bottom surface (11) is flat.

5. A cushion according to one of claims 1 to 3 characterised in that the bottom surface (11) comprises at least two surface portions (57, 58) of which a first surface portion (57) which is towards the body recess (59) extends approximately horizontally and the adjoining second surface portion (58) is disposed remote from the body and is inclined upwardly at an obtuse angle towards the support surface (1).

6. A cushion according to one of claims 1 to 4 characterised in that the upper edge (55) forms the upper body edge (60) in a part of the body recess (59), which is the rear part when the cushion is being worn.

7. A cushion according to one of claims 1 to 6 characterised in that in its part which is the rear part when the cushion is being worn the body recess (59) is defined by the surface of the cylinder of smaller diameter and in its front part it is defined by the surface of the cylinder of larger diameter.

8. A cushion according to one of claims 1 to 7 characterised in that the part of the body recess (59), which is the rear part when the cushion is being worn, blends with a steady increase in the radius of its curvature into the front part.

9. A cushion according to one of claims 1 to 8 characterised in that the waist curve (6) terminates at a rear edge (62) which is remote from the abdominal curve (5) and the upper body edge (60) extends from the rear edge (62) approximately a third of its length in the region of the waist curve (6) and thereafter leads continuously into the abdominal curve (5).

10. A cushion according to one of claims 1 to 9 characterised in that the rear edge (62) defines an end face (10) which is at the rear when the cushion is being worn and which with the support surface (1) forms a transverse edge (63) which is a rear edge when the cushion is being worn while with the bottom surface (11) it forms a lower edge (64) which is a rear edge when the cushion is being worn.

11. A cushion according to one of claims 1 to 10 characterised in that the rear transverse edge (63) and the rear edge (59) include an angle in the range between 20 and 70°.

12. A cushion according to claim 10 or claim 11 characterised in that on its side which is opposite to the end face (10) which is at the rear when the cushion is being worn, the support surface (1) is defined by an end face (9) which is at the front when the cushion is being worn and which is defined by a front edge (67), that is remote from the waist curve (6), of the abdominal curve (5), a front transverse edge (68) which defines the support surface (1) at the front, and a front lower edge (54) which defines the bottom surface (11) at the front, that in the region of the lower edge (54) defining the support surface (1) the front end face (9) firstly extends in a first surface portion (70) in approximately plane-parallel relationship with the rear end face (10) towards the abdominal curve (5) and approximately after a third of the front transverse edge (68) has an inclination in a direction away from the rear end face (10) and forms a second surface portion (71), that the upper edge (55) in the region of the abdominal curve (5) extends in the direction of a straight line deviating therefrom which extends from the transition of the waist curve (6) into the abdominal curve (5) towards that point of the front transverse edge (68) at which the first surface portion (70) goes into the second surface portion (71), that the straight line, with an upper edge portion of the front transverse edge (68), said upper edge portion defining the second surface portion (58), and a portion which is separated from the straight line in the region of the abdominal curve (5) by the upper body edge (60), defines a terminal surface (8) which is inclined downwardly relative to the support surface (1) in a direction towards the bottom surface (11), and that the upper edge portion is at an inclination of about 15° to 30° relative to a lower edge portion of the front transverse edge (68), said lower edge portion extending towards the lower edge (54) of the support surface (1).

13. A cushion according to claim 12 characterised in that the inclination is 22°.

14. A cushion according to one of claims 1 to 13 characterised in that the abdominal curve (5) has a radius in the range of from about 15 cm to 20 cm.

15. A cushion according to claim 14 characterised in that the radius of the abdominal curve is about 32 cm.

16. A cushion according to one of claims 1 to 15 characterised in that the waist curve (6) has a radius in the range of between 10 cm and 20 cm.

17. A cushion according to claim 16 characterised in that the radius of the waist curve (6) is about 15 cm.

18. A cushion according to one of claims 1 to 17 characterised in that it has at least one shoulder strap (20) which in the region of a first end (77) joins an abdominal strap (30) and which in the region of its other end (83) is connected to the cushion body (49).

19. A cushion according to claim 18 characterised in that the shoulder strap (20) is connected releasably to one of the surfaces (1, 9, 10) of the cushion body (49) and fixedly to the abdominal strap (13).

20. A cushion according to one of claims 18 and 19 characterised in that the abdominal strap (13) extends substantially in a horizontal plane.

21. A cushion according to claim 20 characterised in that the abdominal strap (13) joins the waist curve (6) and is fixedly connected thereto along a line (79) which extends substantially parallel to the rear edge (62) and is releasably fixed to the end face (9) which is at the front when the cushion is being worn.

22. A cushion according to claim 21 characterised in that the abdominal strap (13) is fixed to the first surface portion (70) of the front end face (9) by way of a hook-and-loop closure (81) which extends over a large area on the first surface portion (70).

23. A cushion according to one of claims 18 to 2₂ characterised in that in the region of the transition from the back portion (14) into the waist portion the shoulder strap (20) joins the back portion (14) in an approximately perpendicular direction.

24. A cushion according to one of claims 18 to 23 characterised in that the shoulder strap (20) is releasably connected to the support surface (1).

25. A cushion according to claim 24 characterised in that a coupling means which receives the releasable end (80) of the shoulder strap (20) extends on the support surface (1) along the front transverse edge (68).

26. A cushion according to one of claims 23 to 25 characterised in that the shoulder strap (20) extends over a shoulder (21) which is remote from an arm (40) supported on the support surface (1).

27. A cushion according to one of claims 23 to 26 characterised in that the shoulder strap (20) affords a wide surface.

28. A cushion according to one of claims 1 to 27 characterised in that a splint for accommodating the arm (40) is arranged on the support surface (1).

29. A cushion according to claim 28 characterised in that the splint comprises at least one strap (23, 24) for fixing the arm (40).

30. A cushion according to claim 28 or claim 29 characterised in that two straps (23, 24) which extend approximately perpendicularly to each other are disposed on the support surface (1).

31. A cushion according to one of claims 28 to 30 characterised in that an upper arm strap (23) extends over the support surface (1) approximately perpendicularly to the rear transverse edge (63) and a forearm strap (24) extends over the support surface (1) approximately perpendicularly to the lower edge (54).

32. A cushion according to claim 31 characterised in that the upper arm strap (23) is fixed with a fixed end which extends approximately parallel to the rear transverse edge (63) on the support surface (1) in the region of a lower half, which is towards the lower edge (54), of the rear transverse edge (63) in the immediate vicinity thereof.

33. A cushion according to claim 32 characterised in that the upper arm strap (23) is coupled with a loose end (86) that is remote from the fixed end to a coupling (87) which is provided in a central portion of the support surface (1), which central portion is between the upper body edge (60) and the lower edge (54).

34. A cushion according to one of claims 31 to 33 characterised in that the forearm strap (24) is fixed with a fixed end (26) which extends approximately parallel to the lower edge (54) of the support surface (1) on the support surface (1) in the region of a half, which is towards the rear transverse edge (63), of the lower edge (54), in the immediate vicinity thereof.

35. A cushion according to claim 34 characterised in that the forearm strap (24) is coupled with a loose end (28) that is remote from the fixed end (26) to a coupling (87) which is provided in the central portion of the support surface (1).

36. A cushion according to one of claims 33 to 35 characterised in that the forearm strap (24) is coupled to a wide hook-and-loop closure of the upper arm strap (23), said closure forming the coupling (87).

37. A cushion according to one of claims 33 to 35 characterised in that the wide hook-and-loop closure is in the form of a strip which extends transversely over the support surface (1) and whose approximately mutually parallel longitudinal edges (88, 89) extend approximately in a direction from the first surface portion (70) to the rear edge (62).

38. A cushion according to one of claims 32 to 37 characterised in that of the loose ends (28, 86) of the upper arm strap (23) and the forearm strap (24) respectively at least one is provided both on its underside that is towards the hook-and-loop closure which is fixed on the support surface (1) and also on its top side which is opposite to said underside, with a hook-and-loop closure of which a lower one lies on the hook-and-loop closure which is fixed on the support surface (1) and an upper one lies on the hook-and-loop closure which is fixed on the respectively other loose end (28, 86).

39. A cushion according to one of claims 18 to 38 characterised in that the straps (13, 20, 23, 24) are elastic.

40. A cushion according to one of claims 1 to 39 characterised in that the cushion body (49) is formed from a uniform material.

41. A cushion according to one of claims 1 to 40 characterised in that the cushion body (49) is formed from a core (32) and a jacket (33) enclosing the core (32).

42. A cushion according to claim 41 characterised in that the jacket (33) is moisture-resistant.

43. A cushion according to claim 42 characterised in that the jacket (33) is enclosed at least in a region-wise manner by a cover (34).

44. A cushion according to claim 43 characterised in that the cover (34) is in the form of fabric (35).

45. A cushion according to claim 44 characterised in that the fabric (35) is moisture-absorbent at least in the region of the abdominal curve (5).

46. A cushion according to one of claims 41 to 45 characterised in that the core (32) is formed from a material of low specific weight.

47. A cushion according to claim 46 characterised in that the core (32) is in the form of a hollow body (92) enclosing an internal space (91).

48. A cushion according to one of claims 10 to 47 characterised in that a tip (92) which is towards the rear edge (62) is cut off the support surface (1) in the region of the transition of the upper body edge (60) into the rear transverse edge (63).

## Revendications

1. Coussin pour appuyer un bras devant être soutenu en position de repos sur un corps de coussin (49) qui présente au moins une surface d'appui (1) soutenant le bras, au moins une paroi latérale (50) du corps de coussin (49) s'étendant en travers de la surface d'appui (1) et étant constituée d'un évidement (59) dans le corps, qui est délimité par un bord de corps supérieur et un bord de corps inférieur à l'état porté, caractérisé en ce que le bord supérieur (55) de la surface d'appui (1) s'étend au niveau du bord de corps supérieur (60), la surface d'appui (1) s'étendant depuis son bord supérieur (55) dans une direction tournant le dos à l'évidement (59) dans un plan incliné vers le bas en direction du bord inférieur (54) la délimitant, lequel délimite en commun avec le bord inférieur une surface de fond (11), et en ce que l'évidement de corps (59) est composé de surfaces d'au moins deux cylindres dont les axes s'étendent, à l'état porté, à peu près selon la verticale et qui présentent des courbures différentes entre elles, une surface de cylindre présentant un plus petit diamètre étant munie d'une voussure de taille (6) et une surface de cylindre de plus grand diamètre étant munie d'une voussure de ventre (5).

2. Coussin selon la revendication 1, caractérisé en ce que le bord supérieur (55) forme au moins une partie du bord de corps supérieur (60).

3. Coussin selon la revendication 1 ou 2, caractérisé en ce que le bord inférieur (54) limite la surface de fond(11) sur un côté tournant le dos à l'évidement de corps (59).

4. Coussin selon l'une quelconque des revendications 1 à 3, caractérisé en ce que la surface de fond (11) est plate.

5. Coussin selon l'une quelconque des revendications 1 à 3, caractérisé en ce que la surface de fond (11) est constituée par au moins deux surfaces partielles (57,58) parmi lesquelles une première surface partielle (57) tournée vers l'évidement de corps (59) s'étend à peu près horizontalement, et la deuxième surface partielle (58) qui s'y raccorde est placée à distance du corps et est inclinée vers le haut d'un angle obtus dans la direction de la surface d'appui (1).

6. Coussin selon l'une quelconque des revendications 1 à 4, caractérisé en ce que le bord supérieur (55) forme le bord de corps supérieur (60) dans une partie arrière, à l'état porté, de l'évidement de corps (59).

7. Coussin selon l'une quelconque des revendications 1 à 6, caractérisé en ce que l'évidement de corps (59) est délimité dans sa partie arrière à l'état porté par la surface du cylindre de plus petit diamètre et dans sa partie avant par la surface du cylindre de plus grand diamètre.

8. Coussin selon l'une quelconque des revendications 1 à 7, caractérisé en ce que la partie arrière à l'état porté de l'évidement de corps (59) forme une transition avec la partie avant par augmentation continue du rayon de sa courbure.

9. Coussin selon l'une quelconque des revendications 1 à 8, caractérisé en ce que la voussure de taille (6) se termine sur un bord arrière (62) tournant le dos à la voussure de ventre (5), et le bord supérieur (60) s'étend depuis le bord arrière (62) sur environ un tiers de sa longueur au niveau de la voussure de taille (6) puis débouche de manière continue sur la voussure de ventre (5).

10. Coussin selon l'une quelconque des revendications 1 à 9, caractérisé en ce que le bord arrière (62) délimite une surface frontale (10) arrière à l'état porté, qui définit avec la surface d'appui (1) un bord transversal (63) arrière à l'état porté et avec la surface de fond (11) un bord inférieur (64) arrière à l'état porté.

11. Coussin selon l'une quelconque des revendications 1 à 10, caractérisé en ce que le bord transversal arrière (63) et le bord arrière (62) définissent un angle compris entre 20 degrés et 70 degrés.

12. Coussin selon l'une des revendications 10 ou 11, caractérisé en ce que la surface d'appui (1) est délimitée, sur son côté opposé à la surface frontale (10) arrière à l'état porté, par une surface frontale (9) avant à l'état porté, laquelle est délimitée par un bord avant (67) de la voussure de ventre (5) tournant le dos à la voussure de taille (6), par un bord transversal avant (68) délimitant à l'avant la surface d'appui (1) et par un bord inférieur (54) délimitant à l'avant la surface de fond (11), en ce que la surface frontale avant (9), au niveau du bord inférieur (54) délimitant la surface d'appui (1), s'étend d'abord sur une première surface partielle (70) à peu près selon un plan parallèle à la surface frontale arrière (10) dans la direction de la voussure de ventre (5) et, après environ un tiers du bord transversal avant (68) présente une inclinaison dans une direction tournant le dos à la surface frontale arrière (10) et forme une deuxième surface partielle (71), en ce que le bord supérieur (55), au niveau de la voussure de ventre (5), s'étend dans la direction d'une droite s'écartant de celle-ci, qui s'étend depuis la transition entre la voussure de taille (6) et la voussure de ventre (5) dans la direction de la zone du bord transversal avant (68) dans laquelle la première surface partielle (70) se raccorde sur la deuxième surface partielle (71), en ce que la droite, avec le bord partiel supérieur du bord transversal avant (68) qui délimite la deuxième surface partielle (58) et avec une zone partielle de la droite séparée du bord supérieur (60) du corps au niveau de la voussure de ventre (5), délimite une surface de fermeture (8) qui, par rapport à la surface d'appui (1), est inclinée vers le bas en direction de la surface de fond (11), et en ce que le bord partiel supérieur possède une inclinaison d'environ 15 degrés à 30 degrés par rapport à un bord partiel inférieur du bord transversal avant (68) s'étendant dans la direction du bord inférieur (54) de la surface d'appui (1).

13. Coussin selon la revendication 12, caractérisé en ce que l'inclinaison est de 22 degrés.

14. Coussin selon l'une quelconque des revendications 1 à 13, caractérisé en ce que la voussure de ventre (5) présente un rayon compris entre environ 15 cm et 20 cm.

15. Coussin selon la revendication 14, caractérisé en ce que le rayon de la voussure de ventre (5) est d'environ 32 cm.

16. Coussin selon l'une quelconque des revendications 1 à 15, caractérisé en ce que la voussure de taille (6) présente un rayon compris entre environ 10 cm et 20 cm.

17. Coussin selon la revendication 16, caractérisé en ce que le rayon de la voussure de taille (6) est d'environ 15 cm.

18. Coussin selon l'une quelconque des revendications 1 à 17, caractérisé en ce qu'il présente au moins une sangle d'épaule (20) qui se raccorde sur une sangle ventrale (30) au niveau d'une première extrémité (77) et est reliée au corps de coussin (49) au niveau de sa deuxième extrémité (83).

19. Coussin selon la revendication 18, caractérisé en ce que la sangle d'épaule (20) est reliée de manière amovible à l'une des surfaces (1,9,10) du corps de coussin (49) et de manière fixe à la sangle ventrale (13).

20. Coussin selon l'une quelconque des revendications 18 ou 19, caractérisé en ce que la sangle ventrale (13) s'étend pour l'essentiel dans un plan horizontal.

21. Coussin selon la revendication 20, caractérisé en ce que la sangle ventrale (13) se raccorde sur la voussure de taille (6) et est reliée de manière fixe à celle-ci le long d'une ligne (79) qui s'étend de manière approximativement parallèle au bord arrière (62), et est fixée de manière amovible sur la surface frontale (9) avant à l'état porté.

22. Coussin selon la revendication 21, caractérisé en ce que la sangle ventrale (13) est fixée sur la première surface partielle (70) de la surface frontale avant (9) par une bande agrippante (81) s'étendant largement sur la première surface partielle (70).

23. Coussin selon l'une quelconque des revendications 18 à 22, caractérisé en ce que la sangle d'épaule (20) se raccorde à la partie arrière (14) selon une direction approximativement verticale, au niveau de la transition entre la partie arrière (14) et la partie de taille.

24. Coussin selon l'une quelconque des revendications 18 à 23, caractérisé en ce que la sangle d'épaule (20) est liée de manière amovible à la surface d'appui (1).

25. Coussin selon la revendication 24, caractérisé en ce qu'un accouplement accueillant l'extrémité amovible (80) de la sangle d'épaule (20) s'étend sur la surface d'appui (1) le long du bord transversal avant (68).

26. Coussin selon l'une quelconque des revendications 23 à 25, caractérisé en ce que la sangle d'épaule (20) s'étend par-dessus l'épaule (21) du bras opposé au bras (40) reposant sur la surface d'appui (1).

27. Coussin selon l'une quelconque des revendications 23 à 26, caractérisé en ce que la sangle d'épaule (20) est conçue avec une surface large.

28. Coussin selon l'une quelconque des revendications 1 à 27, caractérisé en ce qu'une attelle recevant le bras (40) est placée sur la surface d'appui (1).

29. Coussin selon la revendication 28, caractérisé en ce que l'attelle est constituée d'au moins une sangle (23,24) maintenant en place le bras (40).

30. Coussin selon la revendication 28 ou 29, caractérisé en ce que deux sangles environ perpendiculaires l'une à l'autre (23,24) sont placées sur la surface d'appui (1).

31. Coussin selon l'une quelconque des revendications 28 à 30, caractérisé en ce qu'une sangle de bras (23) s'étend environ à angle droit du bord transversal arrière (63) et une sangle d'avant-bras (24) s'étend environ à angle droit du bord inférieur (54), chacune par-dessus la surface d'appui (1).

32. Coussin selon la revendication 31, caractérisé en ce que la sangle de bras (23) est fixée sur la surface d'appui (1) par une extrémité fixe, qui s'étend environ parallèlement au bord transversal arrière (63), au niveau d'une moitié inférieure du bord transversal arrière (63) tournée vers le bord inférieur (54), et à proximité immédiate de celle-ci.

33. Coussin selon la revendication 32, caractérisé en ce que la sangle de bras (23) est accouplée, par une extrémité libre (86) opposée à l'extrémité fixe, à un accouplement (87) qui est prévu dans une partie intermédiaire de la surface d'appui (1) située entre le bord supérieur du corps (60) et le bord inférieur (54).

34. Coussin selon l'une quelconque des revendications 31 à 33, caractérisé en ce que la sangle d'avant-bras (24) est fixée sur la surface d'appui (1) par une extrémité fixe (26), qui s'étend environ parallèlement au bord inférieur (54) de la surface d'appui (1), au niveau d'une moitié inférieure du bord inférieur (54) tournée vers le bord transversal arrière (63), et à proximité immédiate de celle-ci.

35. Coussin selon la revendication 34, caractérisé en ce que la sangle d'avant-bras (24) est accouplée, par une extrémité libre (28) opposée à l'extrémité fixe (26), à un accouplement (87) qui est prévu dans une partie intermédiaire de la surface d'appui (1).

36. Coussin selon l'une quelconque des revendications 33 à 35, caractérisé en ce que la sangle d'avant-bras (24) est accouplée à la large bande agrippante de la sangle de bras (23) qui constitue l'accouplement (87).

37. Coussin selon l'une quelconque des revendications 33 à 35, caractérisé en ce que la bande agrippante de grande surface est constituée d'une bande s'étendant en travers sur la surface d'appui (1), dont les bords longitudinaux approximativement parallèles entre eux (88,89) s'étendent approximativement dans une direction depuis la première surface partielle (70) vers le bord arrière (62).

38. Coussin selon l'une quelconque des revendications 32 à 37, caractérisé en ce que, des extrémités libres respectives (28,86) de la sangle de bras (23) et de la sangle d'avant-bras (24), au moins l'une est munie d'une bande agrippante aussi bien sur sa face inférieure tournée vers la bande agrippante fixée sur la surface d'appui (1) que sur sa face supérieure en vis-à-vis de la première, celle du dessous reposant sur la bande agrippante fixée sur la surface d'appui (1) et celle du dessus reposant sur la bande agrippante fixée sur l'autre extrémité libre (28,86).

39. Coussin selon l'une quelconque des revendications 18 à 38, caractérisé en ce que les sangles (13,20,23,24) sont élastiques.

40. Coussin selon l'une quelconque des revendications 1 à 39, caractérisé en ce que le corps de coussin (49) est fabriqué dans un seul matériau.

41. Coussin selon l'une quelconque des revendications 1 à 40, caractérisé en ce que le corps de coussin (49) est constitué d'un intérieur (32) et d'une enveloppe (33) entourant l'intérieur (32).

42. Coussin selon la revendication 41, caractérisé en ce que l'enveloppe (33) est constituée pour résister à l'humidité.

43. Coussin selon la revendication 42, caractérisé en ce que l'enveloppe (33) est entourée au moins dans certaines zones d'un dispositif de tension (34).

44. Coussin selon la revendication 43, caractérisé en ce que le dispositif de tension (34) est un tissu (35).

45. Coussin selon la revendication 44, caractérisé en ce que le tissu (35) est constitué pour absorber l'humidité au moins dans la zone de la voussure ventrale (5).

46. Coussin selon l'une quelconque des revendications 41 à 45, caractérisé en ce que l'intérieur (32) est fabriqué dans un matériau de faible densité.

47. Coussin selon la revendication 46, caractérisé en ce que l'intérieur (32) est constitué d'un corps creux (92) enserrant un volume intérieur (91).

48. Coussin selon l'une quelconque des revendications 10 à 47, caractérisé en ce que, dans la zone de transition entre le bord supérieur du corps (60) et le bord transversal arrière (63), une pointe (92) tournée vers le bord arrière (62) est découpée dans la surface d'appui (1).
